# EUROPÄISCHE PATENTSCHRIFT

(11) **EP 3 307 863 B1**
(45) Veröffentlichungstag und Bekanntmachung des Hinweises auf die Patenterteilung: **19.02.2020**
(21) Anmeldenummer: 16717123.0
(22) Anmeldetag: 14.04.2016
(51) Int. Cl.: C12M 1/00

(54) **VORRICHTUNG ZUM SEPARIEREN VON REGENERATIVEN UND ADULTEN STAMMZELLEN**
DEVICE FOR SEPARATING REGENERATIVE AND ADULT STEM CELLS
DISPOSITIF POUR SÉPARER DES CELLULES SOUCHES RÉGÉNÉRATRICES ET ADULTES

(30) Priorität: 10.06.2015 DE 102015106148
(43) Veröffentlichungstag der Anmeldung: 18.04.2018
(73) Patentinhaber: Human Med AG, 19061 Schwerin (DE)
(72) Erfinder: MATTHIESEN, Inge, 23919 Behlendorf (DE); WINKLER, Konrad-Wenzel, 19417 Warin (DE)
(74) Vertreter: Gulde & Partner
(86) Internationale Anmeldenummer: PCT/EP2016/058216
(87) Internationale Veröffentlichungsnummer: WO 2016/198185

(56) Entgegenhaltungen:
- EP-A1- 2 674 479
- WO-A2-2006/127007
- DE-A1-102011 080 218
- DE-A1-102013 209 718

## Beschreibung

Die vorliegende Erfindung betrifft eine Vorrichtung zum Separieren von regenerativen und/oder adulten Stammzellen aus einem einer biologischen Struktur entnommenen Fettgewebe.

Es ist bekannt, Fettgewebe aus biologischen Strukturen, beispielsweise in der Humanmedizin, zu gewinnen. Fettgewebe wird bekanntermaßen durch geeignete Absaugeinrichtungen abgesaugt und in so genannten Fettgewebezellensammlern gesammelt.

Bekannt ist, dass das so gewonnene Fettgewebe neben Fettzellen auch Stammzellen umfasst. Stammzellen sind Körperzellen, die sich in verschiedene Zelltypen oder Gewebe ausdifferenzieren können. Bei den im Fettgewebe vorhandenen Stammzellen handelt es sich um so genannte regenerative sowie adulte Stammzellen.

Eine Gewinnung adulter Stammzellen aus Fettgewebe ist beispielsweise aus der WO 2010/073808 A1 bekannt. Dabei wird das Fettgewebe mit einem Enzym enthaltenden Fluid versetzt und das Gemisch anschließend in einem Behälter zentrifugiert. Hierdurch erfolgt ein Lösen der Stammzellen von den Fettzellen, so dass diese gesondert gesammelt werden können.

Das Separieren kann mittels mechanischer Einrichtungen, zum Beispiel einem Fluidstrahl, wie dies beispielsweise aus DE 10 2011 080 218 A1 bekannt ist, erfolgen.

Aus der DE 10 2013 209 718 A1 ist eine Vorrichtung zum Separieren von adulten Stammzellen bekannt, die gattungsbildend für die vorliegende Erfindung ist.

Unter Verwendung derartiger bekannter Vorrichtungen zum Separieren von Stammzellen ist ein hoher Zeitaufwand, unter anderem bedingt durch eine Vielzahl an Vor- und Zwischenschritten sowie für Reaktionsabläufe, erforderlich.

Es ist daher Aufgabe der vorliegenden Erfindung, eine gattungsgemäße Vorrichtung zum Separieren von Stammzellen zu verbessern. Insbesondere soll es mit der Vorrichtung möglich sein, einen zeitlichen Aufwand zum Separieren der Stammzellen von einem dem Patienten entnommenen Stoffgemisch bis hin zum Vorliegen einer Weiterverwendbarkeit der Stammzellen zu minimieren.

Die Aufgabe wird durch die Gegenstände der unabhängigen Patentansprüche 1 und 8 gelöst.

Die Erfindung betrifft eine Vorrichtung gemäß unabhängigem Anspruch 1 zum Separieren von regenerativen und/oder adulten Stammzellen aus einem einer biologischen Struktur entnommenen Fettgewebe, aufweisend einen Behälter zur Aufnahme eines Stoffgemischs, welches das Fettgewebe und die regenerativen und/oder adulten Stammzellen umfasst, der wenigstens zwei Kammern aufweist, die durch wenigstens eine Membran voneinander getrennt sind. Ferner weist die Vorrichtung eine Stoffgemischzuführeinrichtung auf, die ausgebildet ist, das Stoffgemisch in den Behälter einzuleiten, sowie wenigstens eine Spülmittelzuführeinrichtung, die ausgebildet ist, ein Fluid in den Behälter einzuleiten, sowie eine Stammzellenentnahmeeinrichtung, die ausgebildet ist, die separierten Stammzellen dem Behälter zu entnehmen und die von der Stoffgemischzuführeinrichtung durch die wenigstens eine Membran getrennt ist. Ferner weist die Vorrichtung wenigstens eine Spülmittelabführeinrichtung auf, die ausgebildet ist, ein um regenerative und/oder adulte Stammzellen reduziertes Stoffgemisch aus dem Behälter abzuführen, sowie wenigstens eine spezifisch durchlässige Membran, die für jeweils nur einen spezifischen Teil des jeweiligen Stoffgemischs durchlässig ist. Erfindungsgemäß ist vorgesehen, dass eine erste Kammer des Behälters Mittel zur Separation des Stoffgemischs und einer mit dem Stoffgemisch vermengten Flüssigkeit aufweist.

Eine Vorbehandlung des Stoffgemischs ist vorteilhafterweise nicht mehr erforderlich, da mit dem Stoffgemisch vermengte Flüssigkeiten direkt der Vorrichtung zugeführt werden können Somit kann eine Separation der Stammzellen besonders schnell erfolgen. Es ergeben sich vielfältige weitere Vorteile. So ist die erfindungsgemäße Vorrichtung besonders flexibel. Weiterhin sind Qualität und Ausbeute der Stammzellen besonders gut. Es entfallen auch Risiken eines Stoffgemischverlustes oder einer Verunreinigung infolge eines vorgelagerten Einsatzes konventioneller Vorrichtungen. Ferner sind durch die erfindungsgemäße Vorrichtung konventionelle Vorrichtungen zur Aufbereitung des Stoffgemischs ersetzbar, wodurch der finanzielle und räumliche Aufwand reduziert wird.

Das Mittel zur Separation des Stoffgemischs ist eine Röhre mit einer Druckausgleichsverbindung zu der ersten Kammer sowie ein mit einer Unterdruckquelle verbundenes Saugelement welches in der Röhre angeordnet ist.

Dies bietet den Vorteil, dass eine Trennung von beispielsweise Fettgewebe und anderen Flüssigkeiten in besonders einfacher und schonender Weise erfolgt.

Ein weiterer Aspekt der vorliegenden Erfindung betrifft eine Vorrichtung zum Separieren von regenerativen und/oder adulten Stammzellen aus einem einer biologischen Struktur entnommenen Fettgewebe, aufweisend einen Behälter zur Aufnahme eines Stoffgemischs, welches das Fettgewebe und die regenerativen und/oder adulten Stammzellen umfasst, der wenigstens zwei Kammern aufweist, die durch wenigstens eine Membran voneinander getrennt sind. Ferner weist die Vorrichtung eine Stoffgemischzuführeinrichtung auf, die ausgebildet ist, das Stoffgemisch in den Behälter einzuleiten, sowie wenigstens eine Spülmittelzuführeinrichtung, die ausgebildet ist, ein Fluid in den Behälter einzuleiten, sowie eine Stammzellenentnahmeeinrichtung, die ausgebildet ist, die separierten Stammzellen dem Behälter zu entnehmen und die von der Stoffgemischzuführeinrichtung durch die wenigstens eine Membran getrennt ist. Ferner weist die Vorrichtung wenigstens eine Spülmittelabführeinrichtung auf, die ausgebildet ist, ein um regenerative und/oder adulte Stammzellen reduziertes Stoffgemisch aus dem Behälter abzuführen, sowie wenigstens eine spezifisch durchlässige Membran, die für jeweils nur einen spezifischen Teil des jeweiligen Stoffgemischs durchlässig ist. Erfindungsgemäß ist vorgesehen, dass die wenigstens eine Kammer Mittel zur Temperatureinstellung aufweist.

Dies bietet den Vorteil, dass biologische und/oder chemische Prozesse stets in einem optimalen Temperaturbereich und somit besonders schnell ablaufen können. Somit wird eine Prozesszeit vorteilhaft reduziert. Zudem wird die Qualität des Ergebnisses vorteilhaft verbessert.

In bevorzugter Ausgestaltung der vorliegenden Erfindung ist vorgesehen, dass die Mittel zur Temperatureinstellung eine außen an der Kammer angeordnete Heizmanschette oder ein innerhalb der Kammer angeordnetes Heizelement sowie mindestens einen Temperatursensor aufweisen.

Dies bietet den Vorteil, dass die Vorrichtung besonders einfach und kompakt ist und Wärme direkt einer Wärmeeinflusszone zuführbar ist. Ferner bietet dies den Vorteil, dass die Temperatur regelbar ist. Besonders bevorzugt ist wenigstens ein Mittel zur Temperatureinstellung wenigstens in einem Mischelement integriert. So kann die Wärme vorteilhafterweise direkt im Bereich des Stoffgemischs eingebracht werden, welches gleichzeitig durchmischbar ist. Dadurch wird eine Wärmeverteilung besonders homogen und biologische und/oder chemische Prozesse werden weiter verbessert.

Ein weiterer Aspekt der vorliegenden Erfindung betrifft eine Vorrichtung zum Separieren von regenerativen und/oder adulten Stammzellen aus einem einer biologischen Struktur entnommenen Fettgewebe, aufweisend einen Behälter zur Aufnahme eines Stoffgemischs, welches das Fettgewebe und die regenerativen und/oder adulten Stammzellen umfasst, der wenigstens zwei Kammern aufweist, die durch wenigstens eine Membran voneinander getrennt sind. Ferner weist die Vorrichtung eine Stoffgemischzuführeinrichtung auf, die ausgebildet ist, das Stoffgemisch in den Behälter einzuleiten, sowie wenigstens eine Spülmittelzuführeinrichtung, die ausgebildet ist, ein Fluid in den Behälter einzuleiten, sowie eine Stammzellenentnahmeeinrichtung, die ausgebildet ist, die separierten Stammzellen dem Behälter zu entnehmen und die von der Stoffgemischzuführeinrichtung durch die wenigstens eine Membran getrennt ist. Ferner weist die Vorrichtung wenigstens eine Spülmittelabführeinrichtung auf, die ausgebildet ist, ein um regenerative und/oder adulte Stammzellen reduziertes Stoffgemisch aus dem Behälter abzuführen, sowie wenigstens eine spezifisch durchlässige Membran, die für jeweils nur einen spezifischen Teil des jeweiligen Stoffgemischs durchlässig ist. Erfindungsgemäß ist vorgesehen, dass im Bereich einer zu der Stammzellenentnahmeeinrichtung nächstgelegenen Membran Mittel zur Erzeugung einer zumindest anteilig turbulenten und/oder laminaren Strömung in Stoffgemisch und Spülmittel vorgesehen sind.

Dies bietet den Vorteil, dass die Membran nicht mehr durch Stammzellen zugesetzt werden kann. Somit entfallen zwischengelagerte Schritte zur Befreiung der Membran von Zusetzungen. Die Prozesszeit zur Separation der Stammzellen wird vorteilhaft reduziert. Die erzeugbare Strömung kann laminar, turbulent oder anteilig laminar und turbulent sein. Durch eine sich ergebene Anpassbarkeit von Strömungsverhältnissen lässt sich ein Zusetzen der Membran mit Stammzellen besonders effektiv vermeiden.

In bevorzugter Ausgestaltung der vorliegenden Erfindung ist vorgesehen, dass die Mittel zur Erzeugung einer zumindest anteilig turbulenten und/oder laminaren Strömung ein Rührelement oder eine Düse zur Erzeugung eines Fluidstrahls aufweisen. Dies bietet den Vorteil, dass die Strömung besonders einfach, gezielt und schonend erzeugbar ist.

Besonders bevorzugt ist es, dass die Mittel zur Erzeugung einer zumindest anteilig turbulenten und/oder laminaren Strömung ein berührungslos angetriebenes elektromechanisches Rührelement aufweisen. Dies bietet den Vorteil, dass eine Anzahl mechanischer Komponenten reduziert wird. Ferner wird ein Risiko einer Verunreinigung des Bereichs mit den Stammzellen reduziert, da keine Antriebselemente oder Fluidleitungselemente in diesem Bereich erforderlich sind.

Ein weiterer Aspekt der vorliegenden Erfindung betrifft eine Vorrichtung zum Separieren von regenerativen und/oder adulten Stammzellen aus einem einer biologischen Struktur entnommenen Fettgewebe, aufweisend einen Behälter zur Aufnahme eines Stoffgemischs, welches das Fettgewebe und die regenerativen und/oder adulten Stammzellen umfasst, der wenigstens zwei Kammern aufweist, die durch wenigstens eine Membran voneinander getrennt sind. Ferner weist die Vorrichtung eine Stoffgemischzuführeinrichtung auf, die ausgebildet ist, das Stoffgemisch in den Behälter einzuleiten, sowie wenigstens eine Spülmittelzuführeinrichtung, die ausgebildet ist, ein Fluid in den Behälter einzuleiten, sowie eine Stammzellenentnahmeeinrichtung, die ausgebildet ist, die separierten Stammzellen dem Behälter zu entnehmen und die von der Stoffgemischzuführeinrichtung durch die wenigstens eine Membran getrennt ist. Ferner weist die Vorrichtung wenigstens eine Spülmittelabführeinrichtung auf, die ausgebildet ist, ein um regenerative und/oder adulte Stammzellen reduziertes Stoffgemisch aus dem Behälter abzuführen, sowie wenigstens eine spezifisch durchlässige Membran, die für jeweils nur einen spezifischen Teil des jeweiligen Stoffgemischs durchlässig ist. Erfindungsgemäß ist vorgesehen, dass die Spülmittelabführeinrichtung ausgebildet ist, ein Spülmittel unabhängig von dem um regenerative und/oder adulte Stammzellen reduzierten Stoffgemisch erneut zuzuführen und dieses wieder abzuführen.

Dies bietet den Vorteil, dass nachgelagerte Schritte zur Verringerung einer Restkollagenasekonzentration entfallen können, da dies direkt in der Vorrichtung möglich ist. Die Prozesszeit zur Separation der Stammzellen bis zur Weiterverwendbarkeit wird vorteilhaft reduziert. Die Stammzellen sind direkt aus der Vorrichtung weiterverwendbar, beispielsweise in Form einer Rückführung zum Patienten.

In bevorzugter Ausgestaltung der vorliegenden Erfindung ist vorgesehen, dass die Vorrichtung zumindest teilweise aus sterilen Komponenten besteht. Dies bietet den Vorteil, dass vorgelagerte Schritte zur Sterilisation entfallen können. Ferner ist die Vorrichtung dadurch besonders sicher. Vorrichtungen aus sterilen Komponenten bieten die Möglichkeit, die Vorrichtung als Einmalkit auszuführen. Die Vorrichtung ist somit vollständig an einem sterilen Ort herstellbar, wodurch der Aufwand am Verwendungsort reduziert wird.

Ein weiterer Aspekt der vorliegenden Erfindung betrifft ein System, aufweisend eine Vorrichtung gemäß Anspruch 8 zum Separieren von regenerativen und/oder adulten Stammzellen aus einem einer biologischen Struktur entnommenen Fettgewebe sowie ein zentrales Steuergerät. Erfindungsgemäß ist vorgesehen, dass das zentrale Steuergerät über eine mechanische Schnittstelle für die Vorrichtung als Basisbaugruppe mit dieser verbindbar ist und eine Unterdruckquelle und/oder eine Temperatureinstellung und/oder ein Mittel zur Erzeugung einer Strömung und/oder eine Spülmittelfördereinrichtung zu steuern oder zu regeln vermag.

Dies bietet den Vorteil, dass alle Funktionen zentral einstellbar sind oder auch als Programm vordefinierbar sind. Dadurch wird ein zeitlicher Aufwand zur Bedienung der Vorrichtung vorteilhaft reduziert.

Die einzelnen Aspekte der vorliegenden Erfindung sind mit Vorteil miteinander kombinierbar. Weitere bevorzugte Ausgestaltungen ergeben sich aus den Unteransprüchen und der folgenden Beschreibung.

Die Erfindung wird nachfolgend anhand eines Ausführungsbeispiels und dazugehöriger Zeichnungen näher erläutert. Die Figuren zeigen:
- Figur 1: eine schematische Querschnittsansicht einer erfindungsgemäßen Vorrichtung in einer bevorzugten Ausführungsform;
- Figur 2: eine schematische Darstellung einer detaillierten Querschnittsansicht eines Bereichs einer exemplarischen erfindungsgemäßen Vorrichtung mit einem elektromechanischen Rührelement in einer bevorzugten Ausführungsform und
- Figur 3: eine schematische Darstellung einer Querschnittsansicht eines erfindungsgemäßen Systems in einer bevorzugten Ausführungsform.

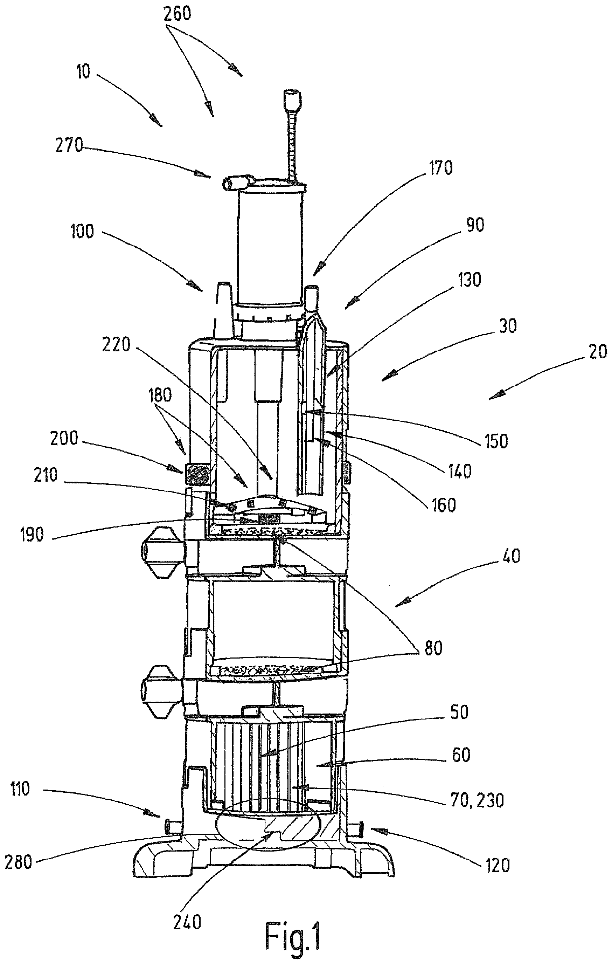

Figur 1 zeigt eine schematische Querschnittsansicht einer erfindungsgemäßen Vorrichtung in einer bevorzugten Ausführungsform. Die erfindungsgemäße Vorrichtung zum Separieren von regenerativen und/oder adulten Stammzellen 10 weist einen Behälter zur Aufnahme eines Stoffgemisches 20 auf. Der Behälter 20 umfasst eine erste Kammer 30, eine zweite Kammer 40, eine dritte Kammer 50 sowie eine vierte Kammer 60. Die dritte Kammer 50 ist von der vierten Kammer 60 durch einen Radialzylinder 70 getrennt. Der Radialzylinder 70 umfasst eine Filtermembran, die ausgebildet ist, die Stammzellen zurückzuhalten. Die erste Kammer 30 ist von der zweiten Kammer 40 und die zweite Kammer 40 von der dritten Kammer 50 ebenfalls durch eine Filtermembran 80 getrennt.

Die erste Kammer 30 weist eine Stoffgemischzuführeinrichtung 90 auf. Die erste Kammer 30 umfasst weiterhin eine Spülmittelzuführeinrichtung 100. An die vierte Kammer 60 schließt sich eine schematisch angedeutete Stammzellenentnahmeeinrichtung 110 an. Ferner schließt sich eine schematisch angedeutete Spülmittelabführeinrichtung 120 an die vierte Kammer 60 an. Die erste Kammer 30 der Vorrichtung 10 verfügt über Mittel zur Separation des Stoffgemisches und einer mit dem Stoffgemisch vermengten Flüssigkeit 130, die systematisch der Stoffgemischzuführeinrichtung 90 zuordenbar sind. Die Mittel 130 umfassen eine Röhre 140 mit einer Druckausgleichsverbindung 150 zu der ersten Kammer 30.

Ferner umfassen die Mittel 130 ein Saugelement 160, das in der Röhre 140 angeordnet ist und an einer Saugseite 170 mit einer hier nicht dargestellten Unterdruckquelle verbunden ist. Die Vorrichtung 10 umfasst weiterhin Mittel zur Temperaturstellung 180. Die Mittel 180 umfassen ein Heizelement 190, eine Heizmanschette 200 und eine Vielzahl an Temperatursensoren 210.

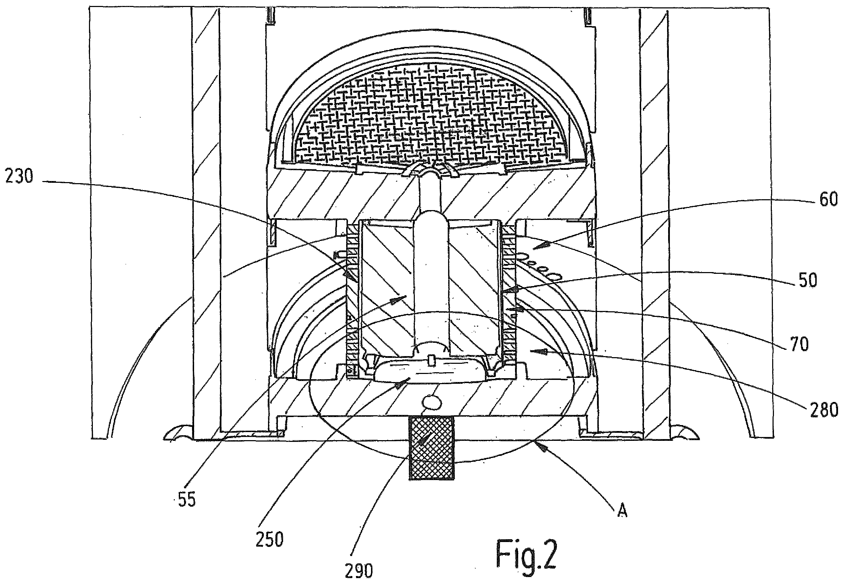

Die Heizmanschette 200 ist außen an der ersten Kammer 30 befestigt. Es können in anderen Ausführungsbeispielen weitere Heizmanschetten 200 außen an weiteren Kammern befestigt sein. Das Heizelement 190 ist in diesem Ausführungsbeispiel in ein Mischelement 220 integriert. Ebenso sind die Temperatursensoren 210 in dem Mischelement 220 integriert. In anderen Ausführungsbeispielen kann die Anordnung und Anzahl der Heizelemente 190 und Temperatursensoren 210 variieren. Der Fachmann nimmt diese Anpassungen nach Maßgabe der Prozessanforderungen vor. Der Radialzylinder 70 umfasst eine zu der Stammzellenentnahmeeinrichtung 110 nächstgelegene Membran 230. Im Bereich der nächstgelegenen Membran 230 sind Mittel zur Erzeugung einer zumindest anteilig turbulenten und/oder laminaren Strömung 240 vorgesehen. Diese umfassen ein berührungslos angetriebenes elektromechanisches Rührelement 250, welches in Figur 2 näher beschrieben wird. Ferner umfassen die Mittel 240 einen Elektromagneten 290 zur Erzeugung eines elektromagnetischen Wechselfeldes, der hier nicht weiter dargestellt ist und in Figur 2 schematisch angedeutet ist. Die Spülmittelabführeinrichtung 120 ist darüber hinaus ausgebildet, unabhängig von dem um adulte und/oder regenerative Stammzellen reduzierten Stoffgemisch erneut ein Spülmittel zuzuführen und dieses wieder abzuführen. Im oberen Teil der Vorrichtung 10 ist ein Anschlussabschnitt 260 vorgesehen, über welchen beispielsweise Kabel für beispielsweise das Heizelement 190 und die Temperatursensoren 210 anschließbar und führbar sind. Ferner ist in diesem Bereich ein Anschluss 270 zur Aufbringung eines Unterdrucks vorgesehen. Die Vorrichtung 10 besteht in solchen Bereichen, die mit dem Stoffgemisch in Berührung kommen, aus sterilen Komponenten. Zu diesen Komponenten zählen beispielsweise die erste Kammer 30, die zweite Kammer 40, die dritte Kammer 50, die vierte Kammer 60, die Stoffgemischzuführeinrichtung 90, die Mittel zur Separation des Stoffgemisches und einer mit dem Stoffgemisch vermengten Flüssigkeit 130, das Mischelement 220, die Filtermembrane 80, der Radialzylinder 70, die Spülmittelabführeinrichtung 120, die Stammzellenentnahmeeinrichtung 110 sowie das hier nicht dargestellte berührungslos angetriebene elektromechanische Rührelement 250. Dieses ist im Bereich A angeordnet und wird in Figur 2 näher beschrieben. Der Bereich A ist in einem unteren Bereich 280 des Radialzylinders 70 angeordnet. Das berührungslos angetriebene elektromechanische Rührelement 250 hat in diesem Ausführungsbeispiel eine kapselartige Form. Der Elektromagnet 290 ist unterhalb des berührungslos angetriebenen elektromechanischen Rührelements 250 angeordnet.

Figur 2 zeigt eine schematische Darstellung einer detaillierten Querschnittsansicht eines Teilbereichs einer erfindungsgemäßen Vorrichtung in einer bevorzugten Ausführungsform. Es gelten dieselben Bezugszeichen wie für Figur 1, so dass für die mit diesen Bezugszeichen referenzierten Merkmale auch das in Figur 1 Beschriebene gilt. Bei dem beschriebenen Teilbereich handelt es sich um einen Teilbereich der in Figur 1 beschriebenen Vorrichtung zum Separieren von regenerativen und adulten Stammzellen 10. Der Teilbereich umfasst unter anderem eine dritte Kammer 50 und eine vierte Kammer 60, einen Bereich A und ein berührungslos angetriebenes elektromechanisches Rührelement 250 sowie den Elektromagneten 290. Ein Volumen der dritten Kammer 50 ist durch ein Kernelement zur Volumenreduktion 55 weitgehend ausgefüllt, so dass lediglich ein schmaler Spalt zwischen dem Kernelement zur Volumenreduktion 55 und der nächstgelegenen Membran 230 frei bleibt. Der Elektromagnet 290 ist unterhalb des berührungslos angetriebenen elektromechanischen Rührelements 250 angeordnet und ausgebildet, ein elektrisches Wechselfeld zu erzeugen, um das berührungslos angetriebene elektromechanische Rührelement 250 zu bewegen. Somit ist innerhalb des Radialzylinders 70 eine anteilig turbulente und anteilig laminare Strömung einstellbar. In anderen Ausführungsbeispielen können auch rein turbulente oder rein laminare Strömungen, beispielsweise mit einer Düse, erzeugt werden.

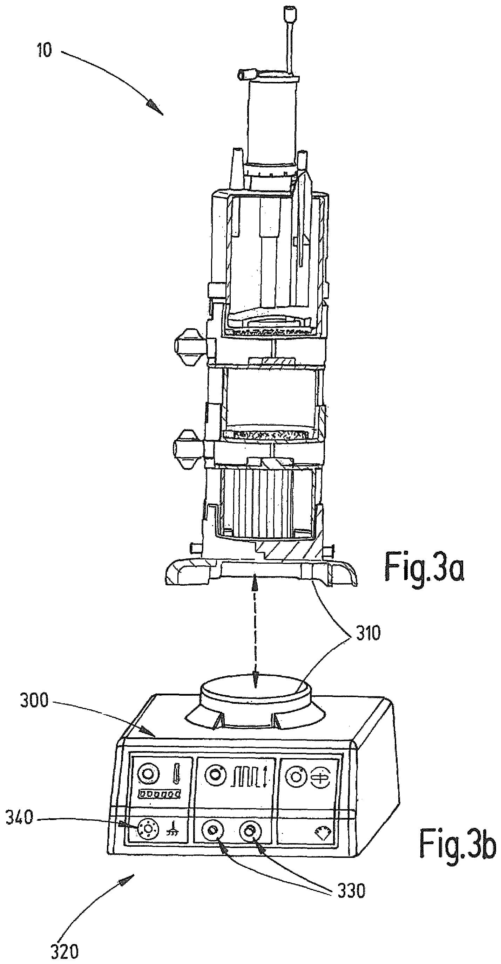

Figur 3 zeigt eine schematische Darstellung einer Querschnittsansicht eines erfindungsgemäßen Systems in einer bevorzugten Ausführungsform. Es gelten dieselben Bezugszeichen wie für Figur 1, so dass für die mit diesen Bezugszeichen referenzierten Merkmale auch das in Figur 1 Beschriebene gilt. Figur 3 zeigt die Vorrichtung zum Separieren von regenerativen und adulten Stammzellen 10 in Verbindung mit einem zentralen Steuergerät 300. Die Vorrichtung zum Separieren von regenerativen und adulten Stammzellen 10 ist in Figur 3a beschrieben. Das zentrale Steuergerät 300 ist in Figur 3b beschrieben. Das zentrale Steuergerät 300 und die Vorrichtung 10 verfügen über eine mechanische Schnittstelle 310, so dass die Vorrichtung 10 auf dem zentralen Steuergerät 300 anordenbar ist. Letzteres verfügt über eine Vielzahl von Anschluss- und Bedienelementen 320. Das zentrale Steuergerät 300 ist ausgebildet, eine Unterdruckquelle, eine Temperatureinstellung, Mittel zur Erzeugung einer Strömung in Form eines durch einen Elektromagneten berührungslos angetriebenen mechanischen Rührelements 250, sowie eine Spülmittelfördereinrichtung zu regeln. Das zentrale Steuergerät 300 verfügt beispielsweise über Anschlüsse für Medien 330 sowie über Anschlüsse für Energie und Signale 340.

### Bezugszeichen

- 10: Vorrichtung zum Separieren von regenerativen und/oder adulten Stammzellen
- 20: Behälter zur Aufnahme eines Stoffgemischs
- 30: erste Kammer
- 40: zweite Kammer
- 50: dritte Kammer
- 55: Kernelement zur Volumenreduktion
- 60: vierte Kammer
- 70: Radialzylinder
- 80: Filtermembrane
- 90: Stoffgemischzuführeinrichtung
- 100: Spülmittelzuführeinrichtung
- 110: Stammzellenentnahmeeinrichtung
- 120: Spülmittelabführeinrichtung
- 130: Mittel zur Separation des Stoffgemischs und einer mit dem Stoffgemisch vermengten Flüssigkeit
- 140: Röhre
- 150: Druckausgleichsverbindung
- 160: Saugelement
- 170: Saugseite
- 180: Mittel zur Temperatureinstellung
- 190: Heizelement
- 200: Heizmanschette
- 210: Temperatursensoren
- 220: Mischelement
- 230: nächstgelegene Membran
- 240: Mittel zur Erzeugung einer zumindest anteilig turbulenten und/oder laminaren Strömung
- 250: berührungslos angetriebenes elektromechanisches Rührelement
- 260: Anschlussabschnitt
- 270: Anschluss
- 280: unterer Bereich
- 290: Elektromagnet
- 300: zentrales Steuergerät
- 310: mechanische Schnittstelle
- 320: Anschluss- und Bedienelemente
- 330: Anschlüsse für Medien
- 340: Anschlüsse für Energie und Signale
- A: Bereich

## Patentansprüche

1. Vorrichtung zum Separieren von regenerativen und/oder adulten Stammzellen (10) aus einem einer biologischen Struktur entnommenen Fettgewebe, aufweisend:
- einen Behälter zur Aufnahme eines Stoffgemischs (20), welches das Fettgewebe und die regenerativen und/oder adulten Stammzellen umfasst, der wenigstens zwei Kammern (30, 40, 50, 60) aufweist, die durch wenigstens eine Membran (80, 70) voneinander getrennt sind;
- eine Stoffgemischzuführeinrichtung (90), die ausgebildet ist, das Stoffgemisch in den Behälter (20) einzuleiten;
- wenigstens eine Spülmittelzuführeinrichtung (100), die ausgebildet ist, ein Fluid in den Behälter (20) einzuleiten;
- eine Stammzellenentnahmeeinrichtung (110), die ausgebildet ist, die separierten Stammzellen dem Behälter (20) zu entnehmen und die von der Stoffgemischzuführeinrichtung (90) durch die wenigstens eine Membran (80, 70) getrennt ist;
- wenigstens eine Spülmittelabführeinrichtung (120), die ausgebildet ist, ein um regenerative und/oder adulte Stammzellen reduziertes Stoffgemisch aus dem Behälter (20) abzuführen und
- wenigstens eine spezifisch durchlässige Membran (80, 70), die für jeweils nur einen spezifischen Teil des jeweiligen Stoffgemischs durchlässig ist,
**dadurch gekennzeichnet, dass**
eine erste Kammer (30) des Behälters (20) Mittel zur Separation des Stoffgemischs und einer mit dem Stoffgemisch vermengten Flüssigkeit (130) aufweist, wobei die Mittel zur Separation des Stoffgemischs (130) eine Röhre (140) mit einer Druckausgleichsverbindung (150) zu der ersten Kammer (30) sowie ein mit einer Unterdruckquelle verbundenes Saugelement (160) aufweisen, welches in der Röhre (140) angeordnet ist.

2. Vorrichtung nach Anspruch 1, **dadurch gekennzeichnet, dass** wenigstens eine Kammer (30, 40, 50, 60) Mittel zur Temperatureinstellung (180) aufweist, wobei die Mittel zur Temperatureinstellung (180) ein innerhalb der Kammer (30, 40, 50, 60) angeordnetes Heizelement (190) sowie mindestens einen Temperatursensor (210) aufweisen.

3. Vorrichtung nach einem der vorhergehenden Ansprüche, **dadurch gekennzeichnet, dass** im Bereich einer zu der Stammzellenentnahmeeinrichtung (110) nächstgelegenen Membran (230) Mittel zur Erzeugung einer zumindest anteilig turbulenten und/oder laminaren Strömung (240) in Stoffgemisch und Spülmittel vorgesehen sind.

4. Vorrichtung nach Anspruch 3, **dadurch gekennzeichnet, dass** die Mittel zur Erzeugung einer zumindest anteilig turbulenten und/oder laminaren Strömung (240) ein Rührelement oder eine Düse zur Erzeugung eines Fluidstrahls aufweisen.

5. Vorrichtung nach Anspruch 3, **dadurch gekennzeichnet, dass** die Mittel zur Erzeugung einer zumindest anteilig turbulenten und/oder laminaren Strömung (240) ein berührungslos angetriebenes elektromechanisches Rührelement (250) aufweisen.

6. Vorrichtung nach einem der vorhergehenden Ansprüche, **dadurch gekennzeichnet, dass** die Spülmittelabführeinrichtung (120) ausgebildet ist, ein Spülmittel unabhängig von dem um adulte Stammzellen reduzierten Stoffgemisch erneut zuzuführen und dieses wieder abzuführen.

7. Vorrichtung nach einem der vorhergehenden Ansprüche, **dadurch gekennzeichnet, dass** die Vorrichtung (10) zumindest teilweise aus sterilen Komponenten besteht.

8. System, aufweisend eine Vorrichtung zum Separieren von regenerativen und/oder adulten Stammzellen (10) aus einem einer biologischen Struktur entnommenen Fettgewebe nach einem der vorhergehenden Ansprüche, sowie ein zentrales Steuergerät (300), **dadurch gekennzeichnet, dass** das zentrale Steuergerät (300) über eine mechanische Schnittstelle (310) für die Vorrichtung (10) als Basisbaugruppe mit dieser verbindbar ist und eine Unterdruckquelle und/oder eine Temperatureinstellung und/oder ein Mittel zur Erzeugung einer Strömung (240) und/oder eine Spülmittelfördereinrichtung zu steuern oder zu regeln vermag.

## Claims

1. A device for separating regenerative and/or adult stem cells (10) from an adipose tissue taken from a biological structure, having:
- a container for holding a material mixture (20), which comprises the adipose tissue and the regenerative and/or adult stem cells, which container has at least two chambers (30, 40, 50, 60) which are separated from each other by at least one membrane (80, 70);
- a material-mixture feeding apparatus (90) which is designed to introduce the material mixture into the container (20);
- at least one flushing-medium feeding apparatus (100) which is designed to introduce a fluid into the container (20);
- a stem cell removal apparatus (110) which is designed to remove the separated stem cells from the container (20) and which is separated from the material-mixture feeding apparatus (90) by the at least one membrane (80, 70);
- at least one flushing-medium draining apparatus (120) which is designed to drain a material mixture, from which regenerative and/or adult stem cells have been removed, from the container (20), and
- at least one specifically permeable membrane (80, 70), each being permeable for only one specific part of the respective material mixture,
**characterised in that**
a first chamber (30) of the container (20) has means for separating the material mixture and a liquid (130) blended with the material mixture, wherein the means for separating the material mixture (130) have a tube (140) with a pressure compensation connection (150) to the first chamber (30) as well as a suction element (160) connected to an underpressure source, said suction element being arranged in the tube (140).

2. The device according to Claim 1, **characterised in that** at least one chamber (30, 40, 50, 60) has means for temperature adjustment (180), wherein the means for temperature adjustment (180) have a heating element (190) arranged within the chamber (30, 40, 50, 60) as well as at least one temperature sensor (210).

3. The device according to any one of the preceding claims, **characterised in that** means for generating an at least proportionately turbulent and/or laminar flow (240) into the material mixture and rinsing agent are provided in the area of a membrane (230) closest to the stem cell removal apparatus (110).

4. The device according to Claim 3, **characterised in that** the means for generating an at least proportionately turbulent and/or laminar flow (240) have a stirring element or a nozzle for generating a fluid jet.

5. The device according to Claim 3, **characterised in that** the means for generating an at least proportionately turbulent and/or laminar flow (240) have a contactlessly-powered electromechanical stirring element (250).

6. The device according to any one of the preceding claims, **characterised in that** the flushing-medium draining apparatus (120) is designed to feed a rinsing agent again independently of the material mixture, from which adult stem cells have been removed, and to drain it again.

7. The device according to any one of the preceding claims, **characterised in that** the device (10) at least partially consists of sterile components.

8. A system, having a device for separating regenerative and/or adult stem cells (10) from an adipose tissue taken from a biological structure according to any one of the preceding claims, as well as a central control appliance (300), **characterised in that** the central control appliance (300) is connectable via a mechanical interface (310) for the device (10) to said device as a basic assembly, and is able to control or regulate an underpressure source and/or a temperature adjustment and/or a means for generating a flow (240) and/or a rinsing agent conveyance apparatus.

## Revendications

1. Dispositif pour la séparation de cellules souches régénératives et/ou adultes (10) à partir d'un tissu graisseux prélevé dans une structure biologique ayant :
- un contenant destiné à la réception d'un mélange de matières (20), qui comprend le tissu graisseux et les cellules souches régénératives et/ou adultes, qui a au moins deux chambres (30, 40, 50, 60), qui sont séparées l'une de l'autre par au moins une membrane (80, 70) ;
- un dispositif d'introduction du mélange de matières (90), qui est conçu pour introduire le mélange de matières dans le contenant (20) ;
- au moins un dispositif d'introduction d'un agent de rinçage (100), qui est conçu pour introduire un fluide dans le contenant (20) ;
- un dispositif de prélèvement de cellules souches (110), qui est conçu pour prélever les cellules souches séparées du contenant (20), et qui est séparé du dispositif d'introduction du mélange de matières (90) par ladite au moins une membrane (80, 70) ;
- au moins un dispositif de déchargement de l'agent de rinçage (120), qui est conçu pour décharger un mélange de matières réduit en cellules souches régénératives et/ou adultes du contenant (20) ; et
- au moins une membrane à perméabilité spécifique (80, 70), qui n'est perméable que pour une partie spécifique du mélange de matières respectif,
**caractérisé en ce que**
une première chambre (30) du contenant (20) a des moyens pour la séparation du mélange de matières et d'un liquide (130) mélangé avec le mélange de matières, les moyens pour la séparation du mélange de matières (130) ayant un tube (140) muni d'un raccordement à égalisation de pression (150) avec la première chambre (30), ainsi qu'un élément d'aspiration (160) raccordé avec une source de sous-pression, qui est agencé dans le tube (140).

2. Dispositif selon la revendication 1, **caractérisé en ce qu'**au moins une chambre (30, 40, 50, 60) a des moyens pour l'ajustement de la température (180), les moyens pour l'ajustement de la température (180) ayant un élément chauffant (190) agencé à l'intérieur de la chambre (30, 40, 50, 60), ainsi qu'au moins un capteur de température (210).

3. Dispositif selon l'une quelconque des revendications précédentes, **caractérisé en ce que,** dans la zone d'une membrane (230) adjacente au dispositif de prélèvement de cellules souches (110), sont prévus des moyens pour la génération d'un écoulement (240) au moins partiellement turbulent et/ou laminaire dans le mélange de matières et l'agent de rinçage.

4. Dispositif selon la revendication 3, **caractérisé en ce que** les moyens pour la génération d'un écoulement (240) au moins partiellement turbulent et/ou laminaire ont un élément d'agitation ou une buse pour la génération d'un jet de fluide.

5. Dispositif selon la revendication 3, **caractérisé en ce que** les moyens pour la génération d'un écoulement (240) au moins partiellement turbulent et/ou laminaire ont un élément d'agitation électromécanique actionné sans contact (250).

6. Dispositif selon l'une quelconque des revendications précédentes, **caractérisé en ce que** le dispositif de déchargement de l'agent de rinçage (120) est conçu pour réintroduire un agent de rinçage indépendamment du mélange de matières réduit en cellules souches adultes et décharger de nouveau celui-ci.

7. Dispositif selon l'une quelconque des revendications précédentes, **caractérisé en ce que** le dispositif (10) est au moins partiellement constitué par des composants stériles.

8. Système, ayant un dispositif pour la séparation de cellules souches régénératives et/ou adultes (10) à partir d'un tissu graisseux prélevé dans une structure biologique selon l'une quelconque des revendications précédentes, ainsi qu'un appareil de commande central (300), **caractérisé en ce que** l'appareil de commande central (300) peut être raccordé avec le dispositif (10) par une interface mécanique (310) pour celui-ci en tant que module de base, et est en mesure de commander ou de réguler une source de sous-pression et/ou un ajustement de température et/ou un moyen pour la génération d'un écoulement (240) et/ou un dispositif de transport d'un agent de rinçage.
